# EUROPÄISCHE PATENTSCHRIFT

(11) **EP 3 088 019 B1**
(45) Veröffentlichungstag und Bekanntmachung des Hinweises auf die Patenterteilung: **14.03.2018**
(21) Anmeldenummer: 16152679.3
(22) Anmeldetag: 26.01.2016
(51) Int. Cl.: A61M 1/36, A61M 39/22, A61M 5/14

(54) **VORRICHTUNG ZUM TRANSFER EINES FLUIDS**
DEVICE FOR TRANSFERRING A FLUID
DISPOSITIF DESTINE A TRANSMETTRE UN FLUIDE

(30) Priorität: 30.04.2015 DE 202015102187 U
(43) Veröffentlichungstag der Anmeldung: 02.11.2016
(73) Patentinhaber: ulrich GmbH & Co. KG, 89081 Ulm (DE)
(72) Erfinder: Koch, Torsten, 89233 Neu-Ulm (DE)
(74) Vertreter: Charrier Rapp & Liebau

(56) Entgegenhaltungen:
- EP-A1- 1 201 264
- WO-A1-93/01859
- WO-A1-2015/136076
- US-A- 5 738 662

## Beschreibung

Die Erfindung betrifft eine Vorrichtung zum Transfer eines Fluids nach dem Oberbegriff des Anspruchs 1, eine Kassette zum Einsetzen in eine Fluidfördervorrichtung, insbesondere in ein Injektions- oder ein Dialysegerät, welche eine solche Vorrichtung umfasst, sowie eine Fluidfördervorrichtung, insbesondere ein Injektions- oder ein Dialysegerät mit einer solchen Kassette.

Eine gattungsgemäße Vorrichtung zum Transfer eines Fluids ist aus der DE 100 53 441 A1 bekannt. Diese beschreibt eine Vorrichtung zur Zuführung und/oder Abzweigung einer Nebenströmung in eine oder aus einer Hauptströmung einer medizinischen Flüssigkeit. Die Vorrichtung umfasst dabei einen Fluidführungskorpus, in dem ein zu einer Seite offener Hauptkanal und zumindest ein in den Hauptkanal mündender Nebenkanal ausgeformt sind. Die offene Seite des Hauptkanals ist von einer Abdeckfolie abgedeckt und die Mündung des Nebenkanals in den Hauptkanal ist von der Abdeckfolie verschließbar. Zum Verschließen der Mündung des Nebenkanals kann die Abdeckfolie von einem Ventilaktor auf die Mündung des Nebenkanals gepresst werden. Diese gattungsgemäße Vorrichtung kann beispielsweise in einer austauschbaren Kassette zum Einsetzen in eine Fluidfördervorrichtung, wie z.B. ein Injektions- oder ein Dialysegerät, eingesetzt werden. Die Ventilaktoren zum Verschließen der Mündung des Nebenkanals sind dabei in der Fluidfördervorrichtung angeordnet und die Kassette wird darin so eingesetzt, dass die Ventilaktoren an Mündungsstellen zu liegen kommen, an denen ein Nebenkanal in den Hauptkanal mündet, so dass durch eine Betätigung der Ventilaktoren (wechselweise) die Mündungen der Nebenkanäle verschlossen werden können.

Bei den bekannten Transfervorrichtungen besteht das Problem, dass die Abdeckfolie, welche als Folie oder als elastische Membran aus einem Elastomermaterial ausgebildet sein kann, bei einem Unterdruck im Hauptkanal oder einem Nebenkanal auch dann auf die Mündung eines Nebenkanals gezogen werden kann, wenn der zugeordnete Ventilaktor nicht betätigt ist. In der Folge kann es dazu kommen, dass der Nebenkanal ungewollt auch bei nicht betätigtem Ventilaktor aufgrund eines Unterdrucks im Haupt- oder dem Nebenkanal verschlossen wird. In entsprechender Weise kann es auch beim Öffnen von verschlossenen Nebenkanalmündungen zu Problemen kommen, wenn im Bereich des Hauptkanals oder des Nebenkanals ein Unterdruck entsteht. Dies kann beispielsweise bei einem betätigten Ventilaktor und verschlossener Mündung eines Nebenkanals entstehen, wenn die Förderpumpe, mit der das Fluid durch die Kanäle gefördert wird, nach dem Abstellen der Pumpe noch kurzzeitig nachläuft. Bei einem gewollten Öffnen des Dichtsitzes der Abdeckfolie auf einer Mündung eines Nebenkanals durch Betätigen des Ventilaktors in seine Öffnungsstellung kann es vorkommen, dass sich die Abdeckfolie aufgrund des Unterdrucks im Nebenkanal nicht von ihrem Dichtsitz auf der Mündungsfläche des Nebenkanals löst und der Nebenkanal deshalb ungewollt verschlossen bleibt.

Hiervon ausgehend liegt der Erfindung die Aufgabe zugrunde, bei einer gattungsgemäßen Vorrichtung zum Transfer eines Fluids ein zuverlässiges Öffnen und Schließen der Mündung eines Nebenkanals zu gewährleisten, ohne dass es zu einem ungewollten Verschluss der Nebenkanalmündung kommen kann, der beispielsweise durch einen Unterdruck im Nebenkanal oder im Hauptkanal hervorgerufen werden kann.

Diese Aufgabe wird mit einer Vorrichtung mit den Merkmalen des Anspruchs 1 gelöst. Bevorzugte Ausführungsformen der erfindungsgemäßen Vorrichtung zum Transfer eines Fluids sind den abhängigen Ansprüchen zu entnehmen.

Die erfindungsgemäße Vorrichtung umfasst einen Hauptkanal und wenigstens einen an einer Mündung in den Hauptkanal mündenden Nebenkanal, der insbesondere als Zufuhrkanal zur Zufuhr eines Fluids vom Nebenkanal in den Hauptkanal dienen kann. Die erfindungsgemäße Vorrichtung umfasst weiterhin ein flexibles Verschlussmittel zum Verschließen der Mündung des Nebenkanals, wobei das Verschlussmittel durch eine externe Kraft, welche beispielsweise durch einen externen Ventilaktor bereitgestellt werden kann, auf die Mündung des Nebenkanals pressbar ist, um die Nebenkanalmündung fluiddicht zu verschließen. Erfindungsgemäß ist dabei dem oder jedem Nebenkanal wenigstens ein Überstand zugeordnet, welcher im Hauptkanal im Bereich der Mündung des jeweiligen Nebenkanals angeordnet ist und über der Mündung oder zumindest über ein unterstes Niveau der Mündung vorsteht. Unter dem untersten Niveau der Mündung eines Nebenkanals wird dabei der Mündungsbereich des Nebenkanals verstanden, der in Strömungsrichtung des Fluids in dem Nebenkanal als erster (strömungsabwärtigster) Bereich in den Hauptkanal führt. Durch den oder jedem Überstand, wird gewährleistet, dass das flexible Verschlussmittel bei einem Unterdruck im Hauptkanal oder im Nebenkanal nicht ungewollt auf die Mündung des Nebenkanals gezogen werden kann, wenn keine externe Kraft auf das Verschlussmittel einwirkt. Dies wird dadurch erreicht, dass das flexible Verschlussmittel bei fehlender externer Kraft durch einen etwaig im Hauptkanal oder im Nebenkanal vorhandenen Unterdruck nur maximal bis auf die stromabwärtige Oberseite des oder jedes Überstands eines Nebenkanals gezogen wird, ohne dass das flexible Verschlussmittel bis in den Bereich der Mündung des Nebenkanals oder in den Bereich des untersten Niveaus der Mündung gelangen kann. Dadurch bleibt die Mündung des Nebenkanals zumindest im Bereich des untersten Mündungsniveaus geöffnet, selbst wenn das flexible Verschlussmittel durch den im Haupt- oder im Nebenkanal herrschenden Unterdruck entgegen der Strömungsrichtung des Fluids im Nebenkanal in Richtung der Mündung des Nebenkanals gezogen wird. Die Mündung des Nebenkanals kann nur durch eine ausreichend große externe Kraft (vollständig) verschlossen werden, welche das Verschlussmittel zum Verschließen des Nebenkanals in bzw. auf dessen Mündung presst. Dabei wird das flexible Verschlussmittel zweckmäßig sowohl auf den oder jeden Überstand des jeweiligen Nebenkanals als auch auf dessen Mündungsrand gepresst, um einen fluiddichten Verschluss des Nebenkanals zu erzeugen.

Der oder jeder einem Nebenkanal zugeordnete Überstand erweist sich auch bei einem gewollten Öffnen der Mündung des Nebenkanals als vorteilhaft. Falls beispielsweise durch ein Nachlaufen einer Förderpumpe, welche ein Fluid durch die erfindungsgemäße Vorrichtung fördert, nach dem Abstellen der Pumpe noch etwas nachläuft, kann es dazu kommen, dass in dem Hauptkanal ein Unterdruck insbesondere im Strömungsbereich um einen Nebenkanal entsteht. Wenn nun gewollt ein durch Einwirken einer externen Kraft auf das flexible Verschlussmittel verschlossener Nebenkanal durch Abstellen der externen Kraft geöffnet werden soll, unterstützt der oder jeder Überstand dieses Nebenkanals das durch die intrinsische Flexibilität des Verschlussmittels hervorgerufene Ablösen des Verschlussmittels von seinem Dichtsitz auf der Mündung des Nebenkanals. Das flexible Verschlussmittel kann sich damit durch Unterstützung der oder jeder Überstand aufgrund einer durch die elastischen Eigenschaften des Verschlussmittels hervorgerufenen Rückstellkraft leichter vom Dichtsitz auf der Mündung des Nebenkanals lösen, auch wenn ein etwaig vorhandener Unterdruck dieser Öffnungsbewegung des Verschlussmittels entgegenwirkt. Dadurch wird gewährleistet, dass das Öffnen und Schließen des Nebenkanals ausschließlich durch das Lösen bzw. das Einwirken einer externen Kraft auf das flexible Verschlussmittel erfolgt und nicht von einem Unterdruck abhängt, der sich ggf. im Hauptkanal oder im Nebenkanal ausgebildet hat.

Bei dem flexiblen Verschlussmittel handelt es sich beispielsweise um eine flexible Folie oder eine flexible Membran , insbesondere aus einem thermoplastischen Elastomer, welche eine ausreichend hohe Flexibilität und Dehnbarkeit aufweist, um von einer externen Kraft, die beispielsweise von einem beweglichen Ventilaktor bereitgestellt werden kann, auf die Mündung eines Nebenkanals gepresst zu werden. Die elastischen und flexiblen Eigenschaften des Verschlussmittels sind dabei so ausgewählt, dass das Verschlussmittel eine ausreichend hohe intrinsische Rückstellkraft bereitstellt, welche das flexible Verschlussmittel selbsttätig in eine Grundstellung bringt, wenn keine externe Kraft auf das Verschlussmittel einwirkt, wobei in der Grundstellung des Verschlussmittels die Mündung des Nebenkanals geöffnet ist.

In einem Ausführungsbeispiel der erfindungsgemäßen Vorrichtung sind der Hauptkanal und jeder Nebenkanal in einem Korpus ausgeformt und der Hauptkanal weist zumindest an den einer Mündung eines Nebenkanals gegenüberliegenden Stellen eine Öffnung auf, welche von dem flexiblen Verschlussmittel abgedeckt ist. Bei dem Korpus kann es sich beispielsweise um ein spritzgegossenes Kunststoff-Gehäuseteil einer Kassette handeln, welche zum Einsetzen in eine Fluidfördervorrichtung, wie z.B. in Injektion- oder ein Dialysegerät, vorgesehen ist. Dabei ist es möglich, dass der Hauptkanal entlang seiner Längsrichtung zu einer Seite offen ist und an der offenen Seite vollständig von dem flexiblen Verschlussmittel, welches dann flächig beispielsweise in Form einer flexiblen Membran oder Folie ausgebildet ist, abgedeckt ist. Es ist jedoch auch möglich, lediglich in den Bereichen, in denen ein Nebenkanal in den Hauptkanal mündet, eine Öffnung in dem Hauptkanal vorzusehen, welche beispielsweise kreisförmig ausgebildet ist und der Mündung des Nebenkanals gegenüberliegt. Diese Öffnung in dem Hauptkanal ist dabei von einem flexiblen Verschlussmittel, beispielsweise einer scheibenförmigen Membran, abgedeckt, welche durch Einwirken einer externen Kraft auf die Mündung des Nebenkanals gepresst werden kann, um den Nebenkanal zu verschließen.

Bei Einsetzen der Kassette mit der darin ausgebildeten erfindungsgemäßen Vorrichtung in eine Fluidfördervorrichtung, wie z.B. ein Injektions- oder ein Dialysegerät, ist zweckmäßig für jeden Nebenkanal ein beweglicher Ventilaktor in der Fluidfördervorrichtung vorgesehen, welcher die externe Kraft zum Andrücken des Verschlussmittels auf die Mündung des Nebenkanals bereitstellt. Der Ventilaktor ist dabei jeweils zwischen einer Schließstellung und einer Öffnungsstellung beweglich, wobei der Ventilaktor das Verschlussmittel in seiner Schließstellung auf bzw. in die Mündung des Nebenkanals presst. In der Öffnungsstellung des Ventilaktors befindet sich das flexible Verschlussmittel in seiner Grundstellung, in der die Mündung des Nebenkanals geöffnet ist, so dass Fluid von dem Nebenkanal in den Hauptkanal (oder auch in umgekehrter Richtung aus dem Hauptkanal in den Nebenkanal) strömen kann.

In bevorzugten Ausführungsbeispielen der Erfindung sind jedem Nebenkanal der erfindungsgemäßen Vorrichtung wenigstens zwei Überstände zugeordnet. Die Überstände sind dabei zweckmäßig gleichmäßig um die Mündung des jeweiligen Nebenkanals verteilt angeordnet. Die Mündung des oder jedes Nebenkanals kann dabei beispielsweise durch das offene Ende eines zylindrischen Rohrs gebildet sein, welches in den Hauptkanal ragt. Die Längsachse des Hauptkanals und die Längsachsen der Zufuhrkanäle können dabei senkrecht zueinander stehen. Es ist jedoch auch möglich, dass ein Nebenkanal unter einem spitzen Winkel in den Hauptkanal mündet.

Zweckmäßig ist der Hauptkanal im Bereich eines einmündenden Nebenkanals als Ringkanal ausgeformt, welcher ringförmig um eine zylindrische Kanalwandung des Nebenkanals verläuft. Dadurch ist eine strömungsgünstige Ausformung des Hauptkanals und des darin einmündenden Nebenkanals gewährleistet, welche die Strömung des Fluids in dem Hauptkanal nicht behindert. Insbesondere wird eine laminare und weitgehend widerstandsfreie Strömung des Fluids im Hauptkanal gewährleistet. Die Querschnittsform des oder jeden Nebenkanals kann jedoch auch anders ausgebildet sein. Zweckmäßig sind dabei stromlinienförmige Außenkonturen der in den Hauptkanal eingreifenden Kanalwandungen des jeweiligen Nebenkanals, wie sie beispielsweise in der DE 100 53 441 A1 vorgeschlagen werden.

Zur Sicherstellung eines zuverlässigen, fluiddichten Verschlusses eines Nebenkanals mittels des flexiblen Verschlussmittels hat es sich als zweckmäßig erwiesen, die Mündung bzw. das unterste Niveau der Mündung des oder jedes Nebenkanals in den Bereich der Mittelebene des Hauptkanals zu legen.

Der Abstand zwischen der stromabwärtigen Oberseite des oder jedes Überstands und der Mündung bzw. dem untersten Niveau der Mündung des zugeordneten Nebenkanals ist zweckmäßig so ausgebildet, dass das Verschlussmittel bei einem möglichen Unterdruck im Hauptkanal oder im Nebenkanal, der bspw. durch eine Fluidförderpumpe einer Fluidfördervorrichtung erzeugt werden kann, zwar auf der stromabwärtigen Oberseite des oder jedes Überstands, nicht jedoch auf der Mündung des Nebenkanals bzw. dessen unterstem Mündungsniveau aufliegt.

Für die Ausbildung des oder jedes Überstands haben sich stromlinienförmige Außenkonturen als vorteilhaft erwiesen, damit die Strömung im Hauptkanal durch den oder jeden Überstand nicht behindert wird. Insbesondere können die Überstände stift-, säulen-, dom- oder pilzförmig ausgebildet sein. Die einem Nebenkanal zugeordneten Überstände sind dabei zweckmäßig in einem Abstand zum Außendurchmesser der Mündungsöffnung des Nebenkanals angeordnet. Es ist jedoch auch möglich, den oder jeden Überstand eines Nebenkanals auf dem oberen Rand der Wandung dieses Nebenkanals anzuordnen, so dass der oder jeder Überstand unmittelbar an den Außendurchmesser der Mündungsöffnung angrenzt. Eine Anordnung der Überstände eines Nebenkanals im Abstand zum Außendurchmesser der Mündungsöffnung ist allerdings zu bevorzugen, weil dadurch ein besserer Verschluss der Mündungsöffnung gewährleistet werden kann, wenn sich das Verschlussmittel in seiner Verschlussstellung befindet. In der Verschlussstellung liegt das Verschlussmittel dabei sowohl auf der strömungsabwärtigen Oberseite des oder jedes Überstands eines Nebenkanals als auch auf dessen Mündungsrand auf, um einen sicheren und fluiddichten Verschluss des Nebenkanals zu gewährleisten.

Diese und weitere Vorteile der erfindungsgemäßen Vorrichtung sowie Anwendungsbeispiele ergeben sich aus den nachfolgend unter Bezugnahme auf die begleitenden Zeichnungen näher beschriebenen Ausführungsbeispielen, in denen eine erfindungsgemäße Vorrichtung zum Transfer von Kontrastmittellösungen und einer Spüllösung in einem Injektionssystem zum intravenösen Injizieren dieser Lösungen in den menschlichen oder tierischen Körper dargestellt wird. Die erfindungsgemäße Vorrichtung ist dabei Bestandteil einer Kassette zum Einsetzen in eine Injektionsvorrichtung. Die Anwendung der Erfindung ist allerdings nicht auf dieses Ausführungsbeispiel beschränkt, welches lediglich beispielhaft die Merkmale und Vorteile der Erfindung beschreibt. Die Zeichnungen zeigen:
- **Figur 1**:: Perspektivische Darstellung einer Kassette zum Einsetzen in eine Injektionsvorrichtung, wobei die Kassette eine erfindungsgemäße Vorrichtung zum Transfer eines Fluids mit einem Hauptkanal und mehreren darin mündenden Nebenkanälen enthält;
- **Figur 2:**: Detailansicht des Hauptkanals der Kassette von Figur 1 im Bereich eines einmündenden Nebenkanals;
- **Figur 3:**: Perspektivische Schnittdarstellungen der Kassette von Figur 1 im Bereich eines in den Hauptkanal mündenden Nebenkanals mit einer Schnittebene in Querrichtung zum Hauptkanal (Figur 3a) und mit einer Schnittebene in Längsrichtung des Hauptkanals (Figur 3b);
- **Figur 4:**: Detailansicht der Schnittebene des Querschnitts von Figur 3 im Bereich der Mündung des Nebenkanals in einer Vorderansicht auf die Schnittebene (Figur 4a) und in einer perspektivischen Ansicht auf die Schnittebene (Figur 4b) zusammen mit einem in den Figuren 1 - 3 nicht gezeigten Verschlussmittel zum Verschließen des Nebenkanals und einem Ventilaktor zur Erzeugung einer externen Kraft, mit der das Verschlussmittel auf die Mündung des Nebenkanals gepresst werden kann, wobei das Verschlussmittel in einer Grundstellung und der Ventilaktor in einer Öffnungsstellung gezeigt sind;
- **Figur 5:**: Querschnittsdarstellung des Nebenkanals von Figur 4, wobei der Ventilaktor in einer Schließstellung gezeigt ist, in der dieser das Verschlussmittel in eine Verschlussstellung auf die Mündung des Nebenkanals presst;
- **Figur 6:**: Querschnittsdarstellung des Nebenkanals von Figur 4, wobei sich das Verschlussmittel in einer Zwischenstellung zwischen der Öffnungsstellung von Figur 4 und der Verschlussstellung von Figur 5 befindet, während sich der nicht dargestellte Ventilaktor in seiner Öffnungsstellung befindet;
- **Figur 7:**: Darstellung einer Ausführungsform der erfindungsgemäßen Vorrichtung mit zwei Überständen im Bereich eines in den Hauptkanal mündenden Nebenkanals in einer Querschnittsdarstellung (Figur 7a) und einer perspektivischen Schnittdarstellung (Figur 7b);
- **Figur 8:**: Perspektivische Schnittdarstellungen weiterer Ausführungsformen der erfindungsgemäßen Vorrichtung mit vier Überständen im Bereich eines in den Hauptkanal mündenden Nebenkanals (Figuren 8a bis 8d);
- **Figur 9:**: Perspektivische Schnittdarstellungen weiterer Ausführungsformen der erfindungsgemäßen Vorrichtung mit mehreren auf der Kanalwandung eines in den Hauptkanal mündenden Nebenkanals angeordneten Überständen (Figuren 9a bis 9d);

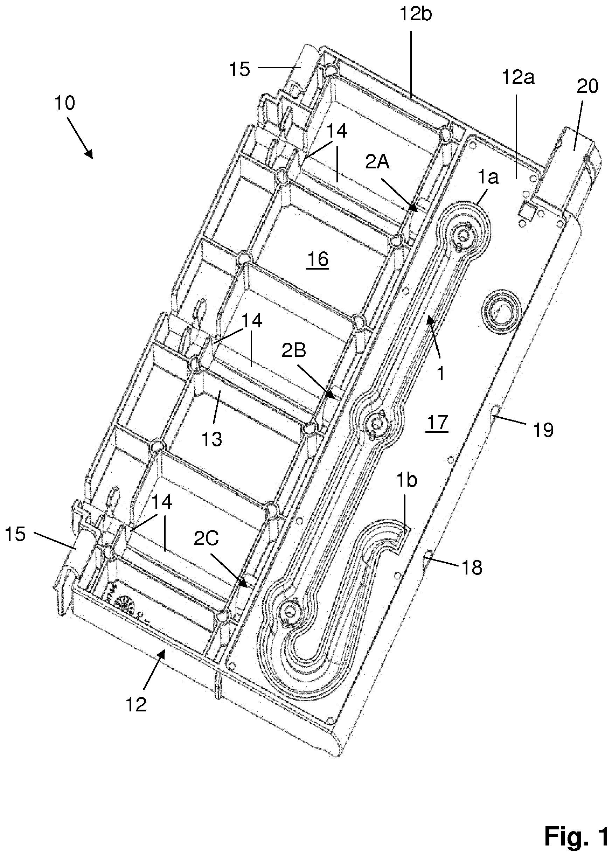

In Figur 1 ist in einer perspektivischen Draufsicht eine Kassette 10 zum Einsetzen in eine hier nicht dargestellte Fluidfördervorrichtung in Form einer Injektionsvorrichtung gezeigt. Die Kassette 10 weist einen Korpus 12 auf, in dem eine erfindungsgemäße Vorrichtung zum Transfer eines Fluids ausgebildet ist. Bei dem Korpus 12 kann es sich beispielsweise um ein spritzgegossenes Kunststoffteil aus einem Hartkunststoff wie PC oder anderen Kunststoffen wie PE oder PP handeln. Der Korpus 12 weist einen Boden 16 auf, an dem vorstehende Seitenwände 13 und Verstärkungsstege 13' angeformt sind. Der Korpus unterteilt sich in einen ersten Abschnitt 12a, in dem die erfindungsgemäße Vorrichtung angeordnet ist, und einen zweiten Abschnitt 12b, in dem mehrere Schlauchaufnahmen 14 zum Einfügen von flexiblen Zufuhrschläuchen vorgesehen sind. Weiterhin ist in dem zweiten Abschnitt 12b des Korpus 12 eine Befestigungseinrichtung 15 zum Befestigen der Kassette 10 in der Injektionsvorrichtung angeordnet.

Im ersten Abschnitt 12a des Korpus 12 sind im Boden 16 Strömungskanäle ausgeformt, nämlich ein Hauptkanal 1, der sich im Wesentlichen in Längsrichtung des ersten Abschnitts 12a des Korpus 12 erstreckt und parallel zum Boden 16 verläuft. Die der Unterseite des Bodens 16 abgewandte Oberseite des Hauptkanals 1 ist offen ausgebildet und wird von einem in Figur 1 nicht gezeigten Verschlussmittel 3 abgedeckt. Bei dem Verschlussmittel 3 handelt es sich um eine flexible Membran oder eine flexible Folie, welche auf der dem Boden 16 abgewandten Oberseite 17 des ersten Korpusabschnitts 12a aufgelegt und dort beispielsweise durch Verkleben oder Verschweißen befestigt wird.

Weiterhin sind in dem Vollmaterial des ersten Abschnitts 12a des Korpus 12 mehrere Nebenkanäle 2 ausgeformt, welche jeweils an einem Ende in den Hauptkanal 1 münden. In dem in Figur 1 dargestellten Ausführungsbeispiel sind drei Nebenkanäle 2A, 2B, 2C vorgesehen. Der am stromaufwärtigen Ende 1a in den Hauptkanal 1 mündende erste Nebenkanal 2A dient zur Zufuhr einer Spüllösung, wie z.B. einer Kochsalzlösung, in den Hauptkanal 1. Hierfür wird das andere Ende des ersten Nebenkanals 2A über einen Zufuhrschlauch mit einem eine Spüllösung enthaltenden Behälter verbunden. Die beiden anderen Nebenkanäle 2B, 2C, welche stromabwärtig in den Hauptkanal 1 münden, dienen der Zufuhr von Kontrastmittellösungen in den Hauptkanal. Hierfür werden die anderen Enden der beiden Nebenkanäle 2B, 2C ebenfalls jeweils über einen Zufuhrschlauch mit einem Behälter verbunden, der eine Kontrastmittellösung enthält. Der Innendurchmesser der Nebenkanäle 2 liegt zweckmäßig im Bereich von 2 bis 4mm und bevorzugt bei ca. 3mm. Der Durchmesser des Hauptkanals 1 ist zweckmäßig etwas größer und liegt bevorzugt im Bereich von 3 bis 6 mm.

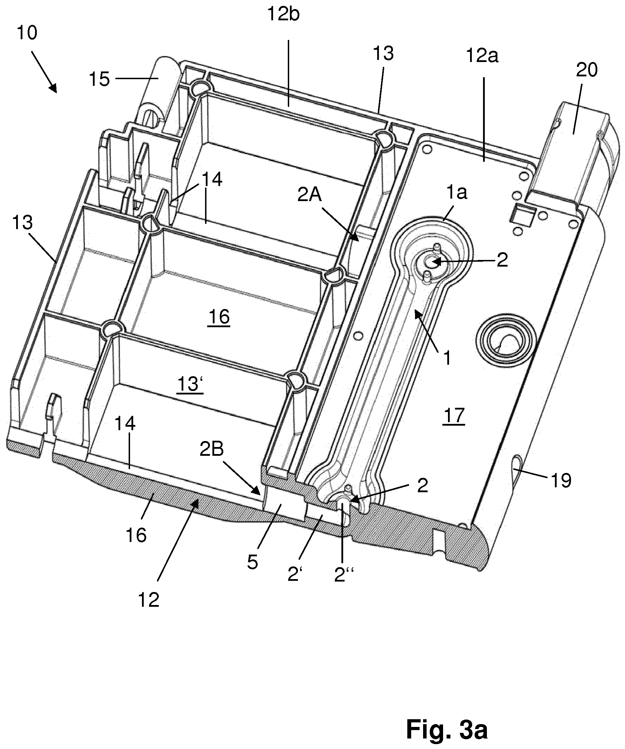

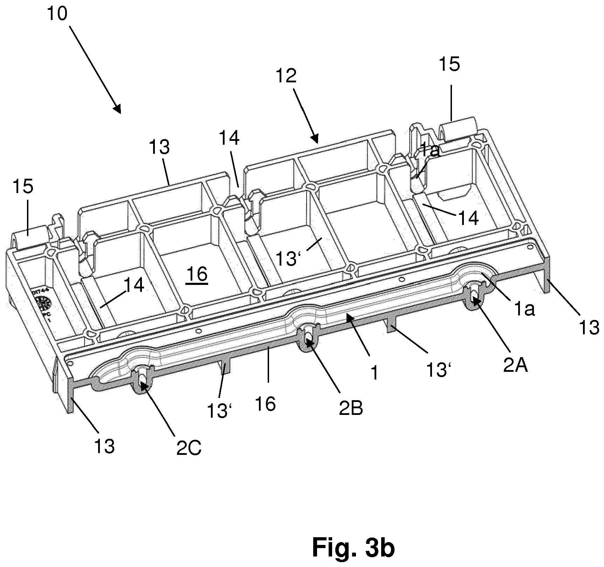

In der Schnittdarstellung von Figur 3 ist in der Schnittebene die Form des Nebenkanals 2B ersichtlich. Dieser weist - wie die übrigen Nebenkanäle 2A, 2C - einen stromaufwärtigen ersten Abschnitt 2' auf, welcher im Wesentlichen parallel zum Boden 16 der Kassette 10 verläuft, sowie einen mit dem ersten Abschnitt 2' verbundenen zweiten Abschnitt 2", der in Form einer senkrecht zum ersten Abschnitt 2' und senkrecht zum Hauptkanal 1 stehenden Bohrung in dem Vollmaterial des Korpus 12 ausgebildet ist und in den Hauptkanal 1 mündet. An dem vom zweiten Abschnitt 2" abgewandten Ende des ersten Abschnitts 2' ist in jedem Nebenkanal 2 ein Anschlussstück 5 angeordnet, welches zum Anschließen eines hier nicht dargestellten Zufuhrschlauchs dient. Ein mit dem Anschlussstück 5 an seinem einen Ende verbundener Zufuhrschlauch wird am anderen Schlauchende mit einem Vorratsbehälter für ein Fluid, beispielsweise eine Kontrastmittellösung oder eine Spüllösung, verbunden.

Am stromabwärtigen Ende 1b des Hauptkanals 1 ist ein Anschluss 18 zum Anschließen eines hier nicht dargestellten Pumpenschlauchs vorgesehen. Ein Ende des Pumpenschlauchs wird für den Betrieb der Kassette 10 mit dem Anschluss 18 verbunden und das andere Ende des Pumpenschlauchs wird mit einem weiteren im ersten Abschnitt 12a des Korpus 12 angeordneten Anschluss 19 verbunden, so dass der Pumpenschlauch schlaufen- oder halbkreisförmig aus dem Korpus 12 der Kassette 10 herausragt. Der Anschluss 19 ist mit einem in dem Korpus ausgeformten und in den Figuren nicht ersichtlichen Abflusskanal verbunden, dessen stromabwärtiges Ende einen Anschluss 20 zum Anschließen eines hier nicht dargestellten Patientenschlauchs aufweist. Der Patientenschlauch wird an einem Ende mit dem Anschluss 20 und am anderen Ende mit einer Kanüle verbunden, welche zur intravenösen Injektion des mit der Kassette 10 transferierten Fluids zur intravenösen Verabreichung der im Hauptkanal 1 geführten Flüssigkeit in eine Vene eines Patienten eingeführt wird.

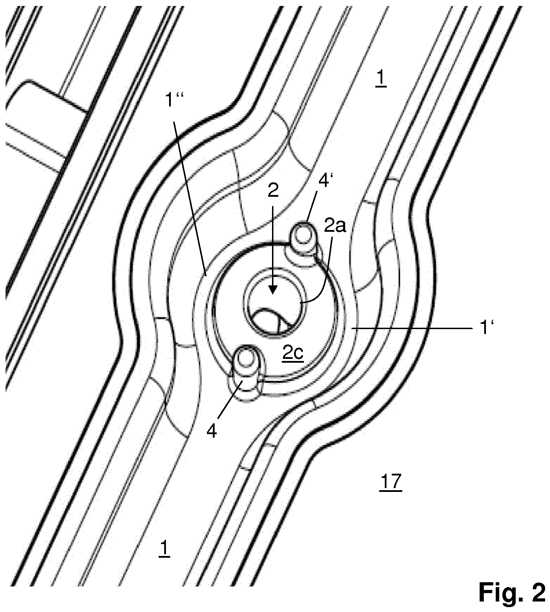

In Figur 2 ist die Mündung 2a eines Nebenkanals 2 in den Hauptkanal 1 im Detail dargestellt. Der zweite Abschnitt 2" dieses Nebenkanals 2, welcher zumindest ungefähr senkrecht zum Hauptkanal 1 verläuft, weist eine rohrförmige Kanalwandung 2c auf, welche in den Hauptkanal 1 mündet. Zweckmäßig liegt die Mündung 2a des Nebenkanals 2, welche durch den oberen Rand der rohrförmigen Kanalwandung 2c gebildet ist, zumindest ungefähr im Bereich einer Mittelebene des Hauptkanals 1. Im Bereich der Mündung 2a des Nebenkanals 2 ist der Hauptkanal 1 als Ringkanal mit zwei Ringkanalabschnitten 1', 1" ausgebildet. Die beiden Ringkanalabschnitte 1', 1" verlaufen dabei ringförmig um die Kanalwandung 2c des Nebenkanals 2.

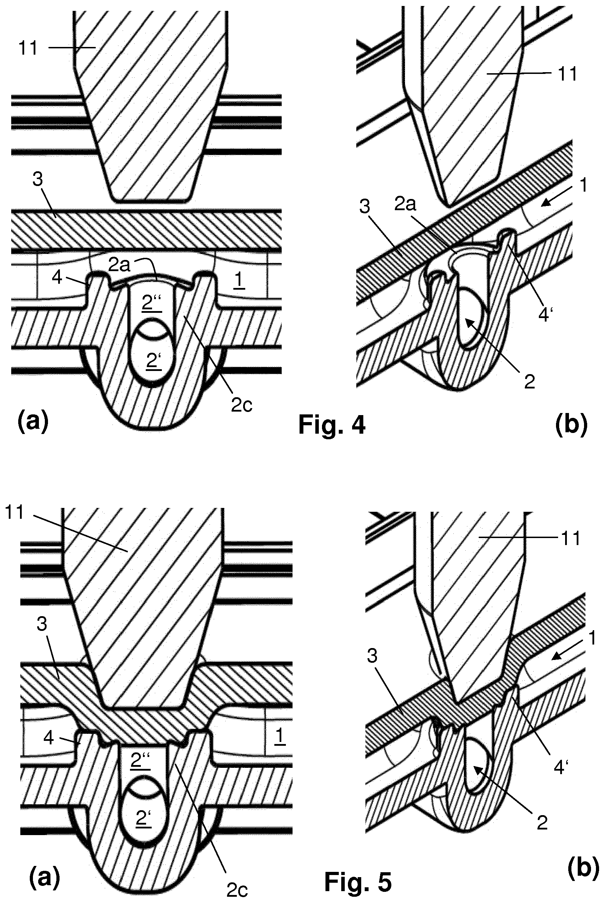

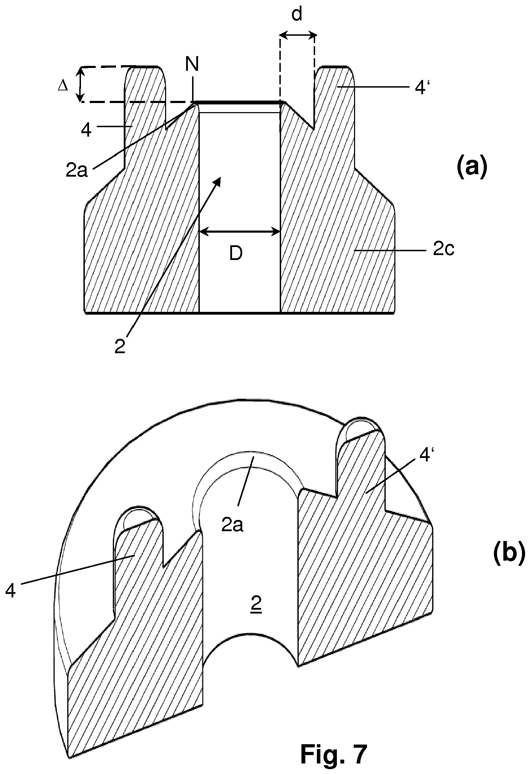

Im Bereich der Kanalwandung 2c des Nebenkanals 2 sind in dem zeichnerisch hier dargestellten Ausführungsbeispiel zwei Überstände 4, 4' angeordnet, welche zylindrisch bzw. dom- oder säulenförmig ausgebildet sind und wie die Kanalwandung 2c in den Hauptkanal 1 hineinragen. Die beiden Überstände 4, 4' sind dabei an diametral gegenüberliegenden Stellen der Mündung 2a angeordnet und stehen über der Mündung 2a des Nebenkanals 2 vor, wie aus den Figuren 4 - 6 ersichtlich. Die Oberseite der beiden Überstände 4, 4' befindet sich damit in Strömungsrichtung des Fluids in dem zweiten Abschnitt 2" des Nebenkanals 2 gesehen im Vergleich zur Mündung 2a auf einem stromabwärtigen Niveau und stehen damit über dem Niveau N der Mündung 2a vor. Dies ist schematisch in Figur 7a angedeutet. Der Überstand zwischen dem Niveau N der Mündung 2a und der Oberseite der Überstände 4, 4'beträgt zweckmäßig zwischen 0,3 und 0,7 mm und liegt bevorzugt bei ca. 0,5 mm.

Die Funktion der erfindungsgemäßen Vorrichtung zum Transfer eines Fluids und insbesondere der Überstände 4, 4' ergibt sich aus den Zeichnungen der Figuren 4 - 6. In den Figuren 4 - 6 ist ein in den Hauptkanal 1 mündender Nebenkanal 2 im Querschnitt zusammen mit dem flexiblen Verschlussmittel 3 zum Verschließen des Nebenkanals 2 und einem Ventilaktor 11 dargestellt. Der Ventilaktor 11 ist dabei Bestandteil einer Fluidfördervorrichtung, bspw. eines Injektors, in welche die Kassette 10 eingesetzt wird. Der Ventilaktor 11 ist dabei zwischen einer in Figur 4 gezeigten Öffnungsstellung und einer in Figur 5 gezeigten Schließstellung beweglich. Zur Bewegung des Ventilaktors 11 zwischen seiner Öffnungsstellung und seiner Schließstellung ist dieser mit einem hier nicht dargestellten Antrieb gekoppelt. Der Ventilaktor 11 dient dazu, das flexible Verschlussmittel 3 zum Verschließen des Nebenkanals 2 auf bzw. in die Mündung 2a des Nebenkanals zu pressen.

In der Darstellung von Figur 4 befindet sich der Ventilaktor 11 in seiner Öffnungsstellung, in der er keine Kraft auf das flexible Verschlussmittel 3 ausübt. Das Verschlussmittel 3 befindet sich entsprechend in einer Grundstellung, in der das Verschlussmittel 3 im Abstand zur Mündung 2a des Nebenkanals 2 angeordnet ist. Der Abstand zwischen der Unterseite des Verschlussmittels 3 und der Mündung 2a des Nebenkanals 2 beträgt in der Grundstellung zweckmäßig zwischen 1 und 2mm und bevorzugt ca. 1,8 mm. Die Mündung 2a des Nebenkanals 2 ist in dieser Grundstellung des Verschlussmittels 3 geöffnet, so dass ein durch den ersten Abschnitt 2' in den Nebenkanal 2 zugeführtes Fluid über den zweiten Abschnitt 2" aus der Mündung 2a des Nebenkanals 2 in den Hauptkanal 1 strömen kann. Das durch das Zusammenwirken des flexiblen Verschlussmittels 3 und des Ventilaktors 11 gebildete Ventil ist in dieser Grundstellung des Verschlussmittels 3 geöffnet.

In Figur 5 ist das Ventil in seiner geschlossenen Stellung gezeigt. Der Ventilaktor 11 befindet sich dabei in seiner Schließstellung, in der er eine externe Kraft auf das flexible Verschlussmittel 3 ausübt. Diese Kraft presst das Verschlussmittel 3 auf die Mündung 2a des Nebenkanals 2, wodurch diese fluiddicht verschlossen wird. Das Verschlussmittel 3 befindet sich in einer Verschlussstellung, in der das Verschlussmittel 3 sowohl auf der Mündung 2a des Nebenkanals 2 (auf dem oberen Rand der Kanalwandung 2c) und gleichzeitig auf der Oberseite der Überstände 4, 4' aufliegt.

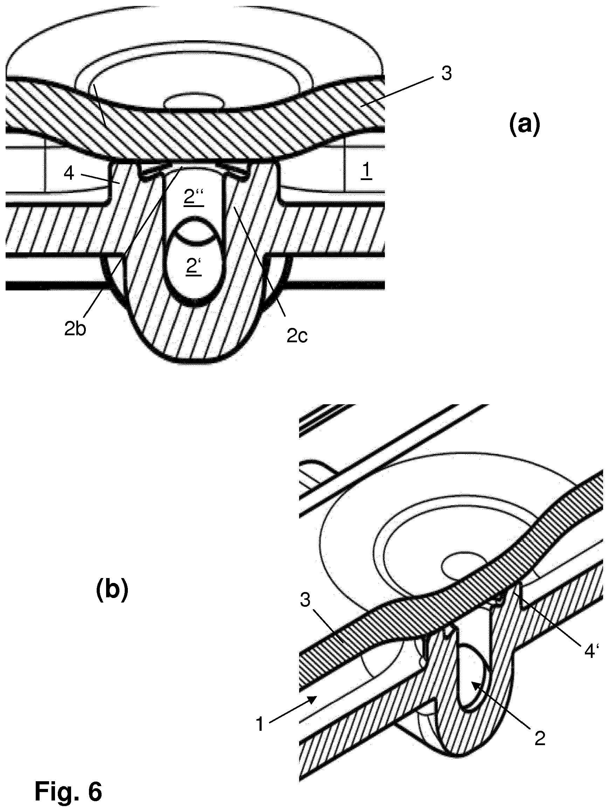

In Figur 6 ist das flexible Verschlussmittel 3 in einer Zwischenstellung zwischen seiner Grundstellung und seiner Verschlussstellung gezeigt. Der in Figur 6 nicht dargestellte Ventilaktor 11 befindet sich dabei in seiner Öffnungsstellung. Die in Figur 6 gezeigte Zwischenstellung wird von dem flexiblen Verschlussmittel 3 beispielsweise dann eingenommen, wenn sich in dem Hauptkanal 1 oder dem Nebenkanal 2 ein Unterdruck ausgebildet hat, der dazu führt, dass das Verschlussmittel 3 aus seiner Grundstellung in Richtung der Mündung 2a des Nebenkanals 2 gezogen wird. Ein sich im Hauptkanal 1 oder im Nebenkanal 2 ausbildender Unterdruck übt eine Zugkraft auf das flexible Verschlussmittel 3 aus, welche dieses in Richtung der Mündung 2a des Nebenkanals zieht. Die durch einen Unterdruck hervorgerufene Zugkraft ist jedoch nicht so groß wie die vom Ventilaktor 11 auf das Verschlussmittel 3 ausgeübte Druckkraft, wenn sich der Ventilaktor 11 in seiner Schließstellung befindet. Das flexible Verschlussmittel 3 wird daher von der Zugkraft nur in die in Figur 6 gezeigte Zwischenstellung gezogen. In dieser Zwischenstellung liegt die Unterseite des flexiblen Verschlussmittels 3 auf der Oberseite der Überstände 4, 4' auf, ohne einen Kontakt mit der Mündung 2a (also der Oberseite der Kanalwandung 2c) des Nebenkanals 2 herzustellen. In dieser Zwischenstellung ist die Mündung 2a des Nebenkanals 2 geöffnet, obwohl auf das flexible Verschlussmittel 3 eine (beispielsweise durch einen Unterdruck hervorgerufene) Zugkraft einwirkt. In diesem Zustand kann bei geöffnetem Ventil ein Fluid aus dem Nebenkanal 2 durch die geöffnete Mündung 2a in den Hauptkanal 1 strömen und es kann ein Druckausgleich zwischen Haupt- und Nebenkanal erfolgen. Dies wird dadurch bewirkt, dass das flexible Verschlussmittel 3 durch die Zugkraft aufgrund der Auflage auf der Oberseite der Überstände 4 nicht bis auf die Mündung 2a des Nebenkanals 2 gezogen werden kann.

Die Überstände 4 sorgen auf diese Weise dafür, dass auch bei Ausbildung eines Unterdrucks im Hauptkanal 1 und/oder im Nebenkanal 2 kein vollständiger Verschluss der Mündung 2a des Nebenkanals 2 erfolgen kann, wenn sich der Ventilaktor 11 in seiner Öffnungsstellung befindet. In umgekehrter Weise sorgen die Überstände 4, 4' beim Öffnen des Ventils durch Bewegung des Ventilaktors 11 von seiner Schließstellung in seine Öffnungsstellung für ein verbessertes Abheben des Verschlussmittels 3 von der Mündung 2a des Nebenkanals 2. Selbst wenn im Hauptkanal 1 oder im Nebenkanal 2 ein Unterdruck herrschen sollte, kann sich das flexible Verschlussmittel 3 aufgrund seiner intrinsischen Elastizität und der dadurch bewirkten elastischen Rückstellkraft selbsttätig in seine Grundstellung bewegen. Diese Bewegung wird dabei von den Überstände 4 unterstützt.

In den Figuren 7a und 7b ist eine bevorzugte Ausführungsform der erfindungsgemäßen Vorrichtung mit zwei Überständen 4, 4' dargestellt, welche an diametral gegenüber liegenden Stellen der Mündung 2a eines Nebenkanals 2 angeordnet sind. Darin befinden sich die beiden Überstände 4, 4' in einem vorgegebenen Abstand d zum äußeren Umfangsrand (am Mündungsdurchmesser D) der Mündung 2a, d.h. die Außenfläche jedes Überstands 4, 4' weist einen vorgegebenen Abstand d zum Mündungsrand auf. Dieser Abstand d liegt zweckmäßig im Bereich von 0,5 bis 2 mm und bevorzugt bei ca. 1,3 mm. Die Höhe Δ des Überstands liegt dabei zweckmäßig im Bereich von 0,2 bis 0,7 mm und bevorzugt bei ca. 0,5 mm (Figur 7a). Die Kanalwandung 2c ist dabei zweckmäßig nach außen hin abfallend abgeschrägt, wie in Figur 7a gezeigt.

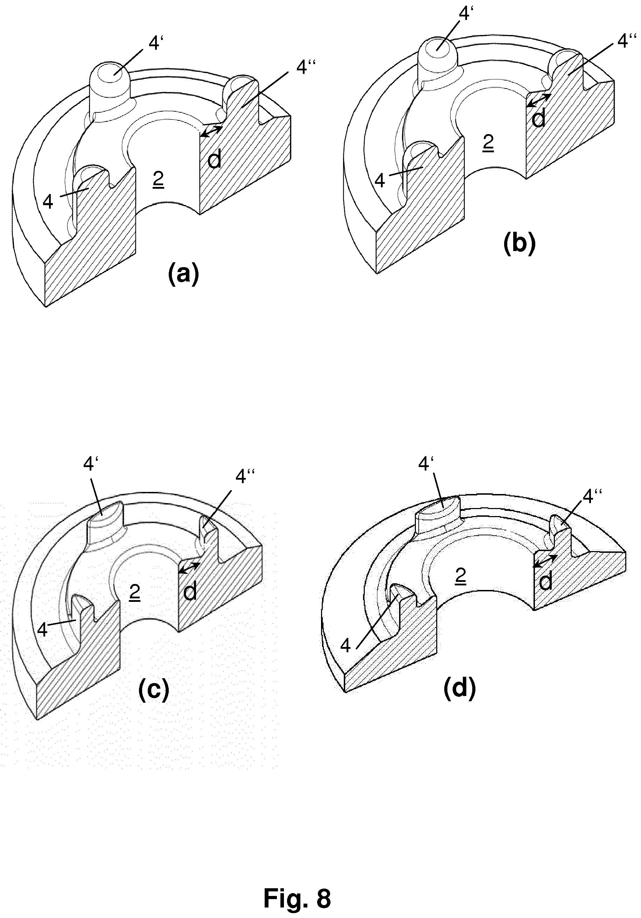

In den Figuren 8a bis 8d sind verschiedene Ausführungsformen der erfindungsgemäßen Vorrichtung mit insgesamt jeweils vier Überständen 4 dargestellt (von denen in der perspektivischen Schnittansicht nur drei Überstände 4, 4', 4" gezeigt sind), welche jeweils unterschiedliche Formen aufweisen und in Umfangsrichtung gleichmäßig (d.h. in Winkelabständen von 90°) um den Mündungsrand verteilt und in den verschiedenen Ausführungsformen der Figuren 8a bis 8d in unterschiedlichen Abständen d zum (radial äußeren) Mündungsrand angeordnet sind.

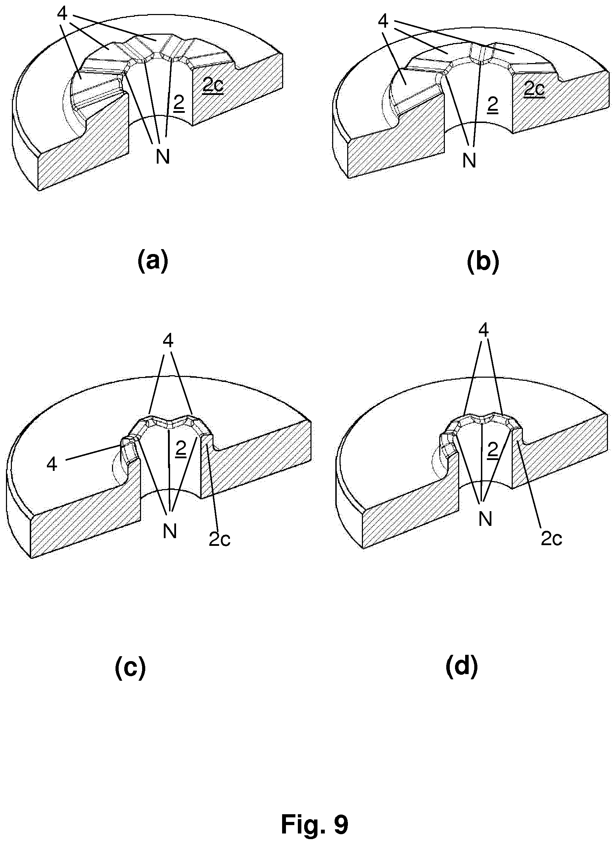

In Figur 9 sind perspektivische Schnittdarstellungen weiterer Ausführungsformen der erfindungsgemäßen Vorrichtung gezeigt (Figuren 9a bis 9d), bei denen mehrere Überstände 4 auf der Kanalwandung 2c eines in den Hauptkanal 1 mündenden Nebenkanals 2 im Abstand zueinander angeordnet sind. In diesen Ausführungsformen schließt der Innenumfang der Überstände 4 bündig mit dem (radial äußeren) Mündungsrand ab, d.h. es besteht kein Abstand zwischen dem Umfangsrand der Mündung 2a und dem Innenumfang des Überstands 4. Jeder der Überstände 4 ist dabei (integral) als Vorsprung auf der Oberseite der Kanalwandung 2c des Nebenkanals 2 ausgeformt. Die Kanalwandung 2c ist dabei zweckmäßig nach außen hin abfallend abgeschrägt, wie in den Figuren 9a bis 9d gezeigt. In den Ausführungsformen der Figuren 9a bis 9d ergibt sich durch die als Vorsprung auf der Oberseite der Kanalwandung 2c des Nebenkanals 2 ausgeformten Überstände 4 ein unterstes Niveau N der Mündung 2a. Als unterstes Mündungsniveau N wird dabei der Bereich der Mündung 2a verstanden, der in Strömungsrichtung des Fluids gesehen als erster in den Hauptkanal 1 mündet. Die Überstände 4 stehen erfindungsgemäß über dem untersten Mündungsniveau N vor, zweckmäßig um ca. 0,3 bis 0,5 mm.

Die Erfindung ist nicht auf das beschriebene Ausführungsbeispiel und den beschriebenen Anwendungsfall beschränkt. Grundsätzlich kann die Erfindung in allen Vorrichtungen zum Transfer von Fluiden eingesetzt werden, in denen das Fluid von einem Nebenkanal in einen Hauptkanal oder auch in umgekehrter Richtung überführt wird und der Nebenkanal mittels einer Quetschventileinrichtung mit einem elastischen Verschlussmittel fluiddicht verschließbar ist. Derartige Vorrichtungen werden bspw. in Injektionsgeräten zur intravenösen Injektion von Flüssigkeiten in den menschlichen oder tierischen Körper oder in Dialysegeräten verwendet. Dabei kann abweichend von dem oben beschriebenen Ausführungsbeispiel nur ein Nebenkanal oder es können auch zwei oder noch mehr Nebenkanäle vorgesehen sein, welche in den Hauptkanal münden. Die Kanäle der erfindungsgemäßen Vorrichtung sind weiterhin nicht zwingend in einem Korpus einer Kassette ausgeformt, sondern können bspw. zumindest abschnittsweise auch als Rohr- oder Schlauchleitungen ausgeführt sein.

## Patentansprüche

1. Vorrichtung zum Transfer eines Fluids, mit einem Hauptkanal (1), wenigstens einem an einer Mündung (2a) in den Hauptkanal (1) mündenden Nebenkanal (2) und einem flexiblen Verschlussmittel (3) zum Verschließen des Nebenkanals (2), wobei die Mündung (2a) des Nebenkanals (2) von dem flexiblen Verschlussmittel (3) fluiddicht verschließbar ist, indem das Verschlussmittel (3) durch eine externe Kraft auf oder in die Mündung (2a) gepresst wird, **dadurch gekennzeichnet, dass** dem oder jedem Nebenkanal (2) wenigstens ein Überstand (4) zugeordnet ist, welcher in dem Hauptkanal (1) im Bereich der Mündung (2a) des jeweiligen Nebenkanals (2) angeordnet ist und über der Mündung (2a) oder über einem untersten Niveau (N) der Mündung (2a) vorsteht.

2. Vorrichtung nach Anspruch 1, wobei das Verschlussmittel (3) so ausgebildet ist, dass es beim Verschließen eines Nebenkanals (2) sowohl auf der Mündung (2a) oder einem untersten Niveau (N) der Mündung (2a) als auch auf dem oder jedem Überstand (4, 4') aufliegt, welcher diesem Nebenkanal (2) zugeordnet ist.

3. Vorrichtung nach Anspruch 1 oder 2, **dadurch gekennzeichnet, dass** es sich bei dem Verschlussmittel (3) um eine flexible Membran, insbesondere aus einem thermoplastischen Elastomer oder um eine flexible Folie handelt, welche mittels eines Ventilaktors auf oder in die Mündung (2a) eines Nebenkanals (2) pressbar ist.

4. Vorrichtung nach einem der voranstehenden Ansprüche, wobei der Hauptkanal (1) und jeder Nebenkanal (2) in einem Korpus (12) ausgeformt sind und der Hauptkanal (1) zumindest an den einer Mündung (2a) eines Nebenkanals (2) gegenüber liegenden Stellen eine Öffnung aufweist, welche von dem flexiblen Verschlussmittel (3) abgedeckt ist.

5. Vorrichtung nach einem der voranstehenden Ansprüche, wobei das Verschlussmittel (3) zwischen einer Grundstellung und einer Verschlussstellung beweglich ist und die Mündung (2a) des Nebenkanals (2) oder ein unterstes Niveau (N) der Mündung (2a) in der Grundstellung offen und in der Verschlussstellung fluiddicht verschlossen ist.

6. Vorrichtung nach einem der voranstehenden Ansprüche, **dadurch gekennzeichnet, dass** jedem Nebenkanal (2) wenigstens zwei Überstände (4, 4') zugeordnet sind, welche bevorzugt gleichmäßig um die Mündung (2a) des jeweiligen Nebenkanals (2) verteilt angeordnet sind.

7. Vorrichtung nach einem der voranstehenden Ansprüche, **dadurch gekennzeichnet, dass** der Hauptkanal (1) im Bereich eines darin einmündenden Nebenkanals (2) als Ringkanal (1', 1") ausgeformt ist, der ringförmig um eine Kanalwandung (2c) des jeweiligen Nebenkanals (2) verläuft.

8. Vorrichtung nach einem der voranstehenden Ansprüche, **dadurch gekennzeichnet, dass** die Mündung (2a) des oder jedes Nebenkanals (2) oder ein unterstes Niveau (N) der Mündung (2a) zumindest ungefähr im Bereich einer Mittelebene des Hauptkanals (1) liegt.

9. Vorrichtung nach einem der voranstehenden Ansprüche, wobei bei einem Unterdruck in dem Hauptkanal (1) und/oder einem der Nebenkanäle (2) das Verschlussmittel (3) zwar auf dem oder jedem Überstand (4, 4') des jeweiligen Nebenkanals (2), nicht jedoch auf dessen Mündung (2a) aufliegt.

10. Vorrichtung nach einem der voranstehenden Ansprüche, **dadurch gekennzeichnet, dass** der oder jeder Überstand (4, 4') zylindrisch, dom-, stift-, säulen- oder pilzförmig ausgebildet ist.

11. Kassette (10) zum Einsetzen in eine Fluidfördervorrichtung, insbesondere in ein Injektions- oder ein Dialysegerät, mit einem Korpus (12), in dem eine Vorrichtung zum Transfer eines Fluids nach einem der voranstehenden Ansprüche ausgeformt ist.

12. Kassette (10) nach Anspruch 11, **dadurch gekennzeichnet, dass** in dem Korpus (12) neben dem Hauptkanal (1) zumindest ein erster Nebenkanal (2A) zur Zuleitung einer Spüllösung und ein zweiter Nebenkanal (2B) zur Zuleitung einer Wirklösung, insbesondere einer Kontrastmittellösung ausgeformt ist.

13. Kassette (10) nach Anspruch 12, **dadurch gekennzeichnet, dass** der erste Nebenkanal (2A) im Bereich des stromaufwärtigen Endes in den Hauptkanal (1) mündet und dass der zweite Nebenkanal (2B) und, sofern vorhanden, jeder weitere Nebenkanal (2C) zur Zuleitung einer Wirklösung weiter stromabwärts in den Hauptkanal (1) mündet.

14. Fluidfördervorrichtung, insbesondere Injektions- oder Dialysegerät, umfassend eine austauschbare Kassette (10) nach einem der Ansprüche 11 bis 13 und wenigstens einen zwischen einer Schließstellung und einer Öffnungsstellung beweglichen Ventilaktor (11), welcher das Verschlussmittel (3) in seiner Schließstellung zum Verschließen eines Nebenkanals (2) auf oder in die Mündung (2a) des Nebenkanals (2) presst.

15. Fluidfördervorrichtung nach Anspruch 14, **dadurch gekennzeichnet, dass** selbst bei einem Unterdruck in dem Hauptkanal (1) und/oder einem der Nebenkanäle (2) das flexible Verschlussmittel (3) durch eine Rückstellkraft des Verschlussmittels (3) von der Mündung (2a) oder einem untersten Niveau (N) der Mündung abgehoben wird, wenn sich der Ventilaktor (11) in seiner Öffnungsstellung befindet.

## Claims

1. Device for transferring a fluid, with a main channel (1), at least one secondary channel (2) leading at an opening (2a) into the main channel (1), and a flexible closing element (3) for closing the secondary channel (2), wherein the opening (2a) of the secondary channel (2) is closable in a fluid-tight manner by the flexible closing element (3) by pressing the closing element (3) with an external force onto or into the opening (2a), **characterized in that** at least one protrusion (4) is associated with each secondary channel (2) and is arranged in the main channel (1) in the area of the opening (2a) of the respective secondary channel (2) and is protruding over the opening (2a) or over a lowest level (N) of the opening (2a).

2. Device according to Claim 1, wherein the closing element (3) is designed in a manner that it bears both on the opening (2a) or a lowest level (N) of the opening (2a) and also on the at least one protrusion (4) associated with this secondary channel (2) during the closing of the secondary channel (2).

3. Device according to Claim 1 or 2, **characterized in that** the closing element (3) is a flexible membrane, in particular made from a thermoplastic elastomer, or a flexible film which can be pressed by a valve actuator onto or into the opening (2a) of a secondary channel (2).

4. Device according to one of the preceding Claims, wherein the main channel (1) and each secondary channel (2) are formed in a body (12) and the main channel (1) has an aperture which is covered by the flexible closing element (3) at least at sites opposite from an opening (2a) of a secondary channel (2).

5. Device according to one of the preceding Claims, wherein the closing element (3) is movable between a base position and a closed position, and the opening (2a) of the secondary channel (2) or a lowest level (N) of the opening (2a) is open in the base position and closed in a fluid-tight manner in the closed position.

6. Device according to one of the preceding Claims, **characterized in that** at least two projections (4, 4') are associated with each secondary channel (2) and preferably are distributed equally around the opening (2a) of the respective secondary channel (2).

7. Device according to one of the preceding Claims, **characterized in that** the main channel (1) is formed in the area of a secondary channel (2) leading therein in the form of a ring channel (1, 1'), which extends in the shape of a ring around a channel wall (2c) of the respective secondary channel (2).

8. Device according to one of the preceding Claims, **characterized in that** the opening (2a) of the or each secondary channel (2) or a lowest level (N) of the opening (2a) is located at least approximately in the area of a centre plane of the main channel (1).

9. Device according to one of the preceding Claims, wherein in the case of a negative pressure in the main channel (1) and/or in one of the secondary channels (2), the closing element (3) bears on the or each protrusion (4) of the respective secondary channel (2), but not against its opening (2a).

10. Device according to one of the preceding Claims, **characterized in that** the or each protrusion (4) is designed cylindrically or in the shape of a dome, a pin, a column or a mushroom.

11. Cassette (10) for insertion into a fluid-conveying device, in particular into an injection apparatus or a dialysis apparatus, with a body (12) in which a device for transferring a fluid is formed according to Claim 1.

12. Cassette (10) according to Claim 11, **characterized in that** in the body (12), in addition to the main channel (1), at least a first secondary channel (2A) for supplying a rinsing solution and a second secondary channel (2B) for supplying an active solution, in particular a contrast agent solution, is formed.

13. Cassette (10) according to Claim 12, wherein the first secondary channel (2A) leads into the main channel in the area of the upstream end, and the second secondary channel (2B) and, if present, each additional secondary channel (2C) for supplying an active solution, leads into the main channel further downstream.

14. Fluid-conveying device, in particular an injection apparatus or a dialysis apparatus, comprising an exchangeable cassette (10) according to one of Claims 11 to 13 and having at least one valve actuator (11) which is movable between a closed position and an open position, and which presses the closing element (3) in its closed position for closing a secondary channel (2) onto or into the opening (2a) of the secondary channel (2).

15. Fluid-conveying device according to Claim 14, **characterized in that** even in the case of a negative pressure in the main channel (1) and/or in one of the secondary channels (2), the flexible closing element (3) is raised by a resetting force of the closing element (3) from the opening (2a) or from a lowest level (N) of the opening, when the valve actuator (11) is in its open position.

## Revendications

1. Dispositif servant au transfert d'un fluide, avec un canal principal (1), au moins un canal secondaire (2) débouchant au niveau d'une embouchure (2a) dans le canal principal (1) et un moyen de fermeture (3) flexible servant à fermer le canal secondaire (2), dans lequel l'embouchure (2a) du canal secondaire (2) peut être fermée de manière étanche aux fluides par le moyen de fermeture (3) flexible, en ce que le moyen de fermeture (3) est pressé par une force externe sur ou dans l'embouchure (2a), **caractérisé en ce qu'**au moins une saillie (4) est associée au canal secondaire ou à chaque canal secondaire (2), laquelle saillie est disposée dans le canal principal (1) dans la zone de l'embouchure (2a) du canal secondaire (2) respectif et dépasse au-delà de l'embouchure (2a) ou au-delà d'un niveau (N) le plus bas de l'embouchure (2a).

2. Dispositif selon la revendication 1, dans lequel le moyen de fermeture (3) est réalisé de telle sorte qu'il repose lors de la fermeture d'un canal secondaire (2) à la fois sur l'embouchure (2a) ou sur un niveau (N) le plus bas de l'embouchure (2a) et sur la saillie ou chaque saillie (4, 4'), qui est associée audit canal secondaire (2).

3. Dispositif selon la revendication 1 ou 2, **caractérisé en ce que** le moyen de fermeture (3) est une membrane flexible, en particulier composée d'un élastomère thermoplastique ou est un film flexible, qui peut être pressé au moyen d'un actionneur de soupape sur ou dans l'embouchure (2a) d'un canal secondaire (2).

4. Dispositif selon l'une quelconque des revendications précédentes, dans lequel le canal principal (1) et chaque canal secondaire (2) sont formés dans un corps (12) et le canal principal (1) présente, au moins en des emplacements faisant face à une embouchure (2a) d'un canal secondaire (2), une ouverture, qui est recouverte par le moyen de fermeture (3) flexible.

5. Dispositif selon l'une quelconque des revendications précédentes, dans lequel le moyen de fermeture (3) est mobile entre une position de base et une position de fermeture et l'embouchure (2a) du canal secondaire (2) ou un niveau (N) le plus bas de l'embouchure (2a) est ouverte/ouvert dans la position de base et est fermée/fermé de manière étanche aux fluides dans la position de fermeture.

6. Dispositif selon l'une quelconque des revendications précédentes, **caractérisé en ce qu'**au moins deux saillies (4, 4') sont associées à chaque canal secondaire (2), lesquelles saillies sont disposées de manière répartie de préférence de manière homogène autour de l'embouchure (2a) du canal secondaire (2) respectif.

7. Dispositif selon l'une quelconque des revendications précédentes, **caractérisé en ce que** le canal principal (1) est formé sous la forme d'un canal annulaire (1', 1'') dans la zone d'un canal secondaire (2) y débouchant, lequel canal annulaire s'étendant de manière à présenter une forme annulaire autour d'une paroi de canal (2c) du canal secondaire (2) respectif.

8. Dispositif selon l'une quelconque des revendications précédentes, **caractérisé en ce que** l'embouchure (2a) du ou de chaque canal secondaire (2) ou un niveau (N) le plus bas de l'embouchure (2a) se situe au moins approximativement dans la zone d'un plan central du canal principal (1).

9. Dispositif selon l'une quelconque des revendications précédentes, dans lequel dans le cas d'une dépression dans le canal principal (1) et/ou dans l'un des canaux secondaires (2), le moyen de fermeture (3) repose certes sur la saillie ou sur chaque saillie (4, 4') du canal secondaire (2) respectif, en revanche pas sur son embouchure (2a).

10. Dispositif selon l'une quelconque des revendications précédentes, **caractérisé en ce que** la saillie ou chaque saillie (4, 4') est réalisée de manière cylindrique, de manière à présenter la forme d'un dôme, d'une tige, d'une colonne ou d'un champignon.

11. Cassette (10) destinée à être insérée dans un dispositif de refoulement de fluide, en particulier dans un appareil d'injection ou dans un appareil de dialyse, avec un corps (12), dans lequel est formé un dispositif servant au transfert d'un fluide selon l'une quelconque des revendications précédentes.

12. Cassette (10) selon la revendication 11, **caractérisée en ce qu'**au moins un premier canal secondaire (2A) servant à acheminer une solution de rinçage et un deuxième canal secondaire (2B) servant à acheminer une solution active, en particulier une solution d'agent contrastant sont formés dans le corps (12) en plus du canal principal (1).

13. Cassette (10) selon la revendication 12, **caractérisée en ce que** le premier canal secondaire (2A) débouche dans la zone de l'extrémité en amont dans le canal principal (1), et que le deuxième canal secondaire (2B) et, si disponible, chaque autre canal secondaire (2C) servant à acheminer une solution active débouchent par ailleurs en aval dans le canal principal (1).

14. Dispositif de refoulement de fluide, en particulier appareil d'injection ou de dialyse, comprenant une cassette (10) remplaçable selon l'une quelconque des revendications 11 à 13 et au moins un actionneur de soupape (11) mobile entre une position de fermeture et une position d'ouverture, lequel presse sur ou dans l'embouchure (2a) du canal secondaire (2) le moyen de fermeture (3) dans sa position de fermeture afin de fermer un canal secondaire (2).

15. Dispositif de refoulement de fluide selon la revendication 14, **caractérisé en ce que** même dans le cas d'une dépression dans le canal principal (1) et/ou dans l'un des canaux secondaires (2), le moyen de fermeture (3) flexible est soulevé par une force de rappel du moyen de fermeture (3) de l'embouchure (2a) ou d'un niveau (N) le plus bas de l'embouchure quand l'actionneur de soupape (11) se trouve dans sa position d'ouverture.
